# EUROPÄISCHE PATENTANMELDUNG

(11) **EP 3 425 413 A1**
(43) Veröffentlichungstag der Anmeldung: **09.01.2019**
(21) Anmeldenummer: 17179594.1
(22) Anmeldetag: 04.07.2017
(51) Int. Cl.: G01R 33/28

(54) **PERMANENTE ÜBERWACHUNG EINES EINE MAGNETRESONANZANLAGE AUFWEISENDEN UNTERSUCHUNGSRAUMS**

(71) Anmelder: Siemens Healthcare GmbH, 91052 Erlangen (DE)
(72) Erfinder: Ferguson, George William, 91056 Erlangen (DE); Nufer, Stephan, 91056 Erlangen (DE); Paul, Dominik, 91088 Bubenreuth (DE)

(57) **Zusammenfassung**

In einem Untersuchungsraum (1) ist eine Magnetresonanzanlage (6) angeordnet, die einen Untersuchungsbereich (7), insbesondere einen Untersuchungstunnel, aufweist. Eine Auswertungseinrichtung (12) nimmt unabhängig vom Betrieb der Magnetresonanzanlage (6) von einem Videokamerasystem (9) aus einem Erfassungsbereich Bilder (B) entgegen. Der Erfassungsbereich umfasst Zugänge (5) zum Untersuchungsraum (7) und/oder einen Bereich (10) vor dem Untersuchungsbereich (7). Immer dann, wenn die Auswertungseinrichtung (12) in einem Bereitschaftszustand (Z1) in den entgegengenommenen Bildern (B) eine Person (15) erkennt, prüft sie, ob eine vom Erkennen einer Person (15) als solches verschiedene Alarmbedingung erfüllt ist. Wenn die Alarmbedingung erfüllt ist, geht sie in einen Alarmzustand (Z2) über, in dem sie zumindest einmalig zumindest ein im Untersuchungsraum (1) hörbares akustisches und/oder sichtbare optisches Signal ausgibt. Anderenfalls behält sie den Bereitschaftszustand (Z1) bei. Im Alarmzustand (Z2) prüft die Auswertungseinrichtung (12), ob ihr über eine Mensch-Maschine-Schnittstelle (18) ein Beendigungsbefehl (T) vorgegeben wird. Bei Vorgabe des Beendigungsbefehls (T) geht sie in einen Abschaltzustand (Z3) über, in dem sie das akustische und/oder optische Signal nicht mehr ausgibt. Im Alarmzustand (Z2) und im Abschaltzustand (Z3) prüft die Auswertungseinrichtung (12), ob sie in den Bildern (B) weiterhin die Person (15) erkennt. Wenn sie weiterhin die Person (15) erkennt, behält sie den momentanen Zustand (Z2, Z3) bei. Anderenfalls geht sie in den Bereitschaftszustand (Z1) über.

## Beschreibung

Die vorliegende Erfindung geht aus von einem Überwachungsverfahren für einen Untersuchungsraum, in dem eine einen Untersuchungsbereich, insbesondere einen Untersuchungstunnel, aufweisende Magnetresonanzanlage angeordnet ist.

Die vorliegende Erfindung geht weiterhin aus von einem Computerprogramm, das Maschinencode umfasst, der von einer Auswertungseinrichtung abarbeitbar ist, wobei die Abarbeitung des Maschinencodes durch die Auswertungseinrichtung bewirkt, dass die Auswertungseinrichtung ein derartiges Überwachungsverfahren ausführt.

Die vorliegende Erfindung geht weiterhin aus von einer Auswertungseinrichtung, wobei die Auswertungseinrichtung mit einem derartigen Computerprogramm programmiert ist, so dass die Auswertungseinrichtung im Betrieb ein derartiges Überwachungsverfahren ausführt.

Die vorliegende Erfindung geht weiterhin aus von einem Untersuchungsraum,
- wobei in dem Untersuchungsraum eine einen Untersuchungsbereich, insbesondere einen Untersuchungstunnel, aufweisende Magnetresonanzanlage angeordnet ist,
- wobei dem Untersuchungsraum ein Videokamerasystem zugeordnet ist, mittels dessen aus einem Erfassungsbereich Bilder erfassbar sind,
- wobei der Erfassungsbereich einen Bereich vor dem Untersuchungsbereich umfasst,
- wobei das Videokamerasystem mit einer Auswertungseinrichtung verbunden ist.

Aus der DE 10 2015 211 148 A1 bzw. aus der korrespondierenden US 2016 0 367 169 A1 ist ein Untersuchungsraum der eingangs genannten Art bekannt. Bei diesem Untersuchungsraum werden im Rahmen der Vorbereitung einer Untersuchung Bilder aus dem Bereich vor dem Untersuchungsbereich erfasst. Eine Person wird anhand der erfassten Bilder lokalisiert. In Abhängigkeit von der erfassten Lokalisierung können Informationen auf bestimmte Körperteile der Person projiziert werden.

Magnetresonanzanlagen weisen oftmals einen supraleitenden Grundmagneten auf, der ein hohes statisches Grundmagnetfeld von beispielsweise 1,5 T oder 3T generiert. Das starke Magnetfeld zieht ferromagnetische Objekte mit einer entsprechend starken Kraft an. Das Magnetfeld kann weiterhin beispielsweise die Funktion eines Herzschrittmachers oder eines anderen Implantats beeinträchtigen. Auch kann es dazu führen, dass ferromagnetische Objekte, die versehentlich in den Bereich des Kraftfeldes gebracht werden, von dem Kraftfeld angezogen werden. Dadurch kann das ferromagnetische Objekt beim Aufschlagen auf Gegenstände oder eine Person Schäden verursachen.

Der Betreiber der Magnetresonanzanlage ist verpflichtet, das Bedienungspersonal der Magnetresonanzanlage entsprechend zu schulen und weiterhin auch entsprechende Warnhinweise an den Zugängen zu dem (geschlossenen) Untersuchungsraum anzubringen, in dem sich die Magnetresonanzanlage befindet. Dennoch kommt es immer wieder zu Unfällen durch ungeschultes Personal oder Patienten oder Angehörige von Patienten, die den Untersuchungsraum betreten und hierbei magnetisierbare Objekte bei sich führen, beispielsweise eine Sauerstoffflasche, ein Beatmungsgerät, einen Rollstuhl oder - im Falle einer Reinigungskraft - beispielsweise ein entsprechendes Reinigungsgerät oder Bohnergerät. Die entsprechenden Warnschilder werden entweder nicht gesehen oder nicht beachtet. In manchen Fällen wird auch trotz Erkennens der Warnschilder die Gefahr unterschätzt. In seltenen Fällen treten derartige Unfälle auch bei prinzipiell geschultem Personal auf, wenn die Schulung schon zu lange zurückliegt.

Die Aufgabe der vorliegenden Erfindung besteht darin, Möglichkeiten zu schaffen, mittels derer derartige gefährliche Situationen und Unfälle zumindest weitestgehend vermieden werden können.

Die Aufgabe wird durch ein Überwachungsverfahren mit den Merkmalen des Anspruchs 1 gelöst. Vorteilhafte Ausgestaltungen des Überwachungsverfahrens sind Gegenstand der abhängigen Ansprüche 2 bis 9.

Erfindungsgemäß wird ein Überwachungsverfahren der eingangs genannten Art geschaffen, bei dem eine Auswertungseinrichtung
- unabhängig vom Betrieb der Magnetresonanzanlage von einem Videokamerasystem aus einem Erfassungsbereich Bilder entgegennimmt, wobei der Erfassungsbereich Zugänge zum Untersuchungsraum und/oder einen Bereich vor dem Untersuchungsbereich umfasst,
- immer dann, wenn sie in einem Bereitschaftszustand in den entgegengenommenen Bildern eine Person erkennt, prüft, ob eine vom Erkennen einer Person als solches verschiedene Alarmbedingung erfüllt ist,
- immer dann, wenn die Alarmbedingung erfüllt ist, in einen Alarmzustand übergeht, in dem sie zumindest einmalig zumindest ein im Untersuchungsraum hörbares akustisches Signal und/oder ein im Untersuchungsraum sichtbares optisches Signal ausgibt, und anderenfalls den Bereitschaftszustand beibehält,
- im Alarmzustand prüft, ob ihr über eine Mensch-Maschine-Schnittstelle ein Beendigungsbefehl vorgegeben wird,
- bei Vorgabe des Beendigungsbefehls in einen Abschaltzustand übergeht, in dem sie das akustische Signal und/oder das optische Signal nicht mehr ausgibt,
- im Alarmzustand und im Abschaltzustand prüft, ob sie in den Bildern weiterhin die Person erkennt,
- immer dann, wenn sie im Alarmzustand und im Abschaltzustand weiterhin die Person erkennt, den momentanen Zustand beibehält und anderenfalls in den Bereitschaftszustand übergeht.

Das Videokamerasystem und die Auswertungseinrichtung werden erfindungsgemäß also nicht nur im Rahmen des Betriebs der Magnetresonanzanlage (also im Rahmen von Untersuchungen und im Vorfeld von derartigen Untersuchungen) eingesetzt, sondern arbeiten quasi kontinuierlich. Die Auswertungseinrichtung prüft zunächst, ob sie überhaupt eine Person erkennt. Das Erkennen einer Person als solches löst aber noch keinen Alarm aus. Ein Alarm wird vielmehr nur dann ausgelöst, wenn zusätzlich eine Alarmbedingung erfüllt ist. Die Alarmbedingung ist dann erfüllt, wenn anhand zusätzlicher Kriterien erkannt wird, dass eine Gefahrensituation vorliegen könnte. In diesem Fall wird ein akustisches Signal und/oder ein optisches Signal ausgegeben, also eine entsprechende Warnung.

In manchen Fällen wird die Warnung ein Fehlalarm sein. Dies ist jedoch unkritisch, da in diesem Fall der Fehlalarm durch Vorgeben des Beendigungsbefehls beendet werden kann.

Die Alarmbedingung kann in verschiedener Weise ausgestaltet sein.

Beispielsweise ist es möglich, dass die Alarmbedingung nur dann erfüllt ist, wenn die Auswertungseinrichtung in den entgegengenommenen Bildern zusätzlich zu der Person an der Person ein von der Person und dessen Bekleidung verschiedenes Objekt erkennt. Das Erkennen des Objekts ist eine notwendige, nicht aber zwangsweise eine hinreichende Bedingung für das Erfüllen der Alarmbedingung. Es ist zwar möglich, dass das Erkennen des Objekts bereits hinreichend ist. Es ist aber nicht zwingend. Beispielsweise ist es möglich, dass die Alarmbedingung nur dann erfüllt ist, wenn das erkannte Objekt eine hinreichende Übereinstimmung mit mindestens einem vorbestimmten Objekttyp aufweist. Die Objekttypen können hierbei nach Bedarf spezifiziert sein. Beispielsweise können die Objekttypen eine Flasche (beispielsweise eine Sauerstoffflasche), einen Stuhl (insbesondere einen Rollstuhl), eine Armbanduhr und/oder eine Brille umfassen. Auch andere Objekttypen wie beispielsweise ein Beatmungsgerät, ein Bett oder ein Patientenbett, ein Schrubber, ein Besen oder ein motorisch betriebenes Reinigungsgerät sind denkbar.

Alternativ oder zusätzlich ist es möglich, dass die Alarmbedingung nur dann erfüllt ist, wenn die erkannte Person sich in einem vorbestimmten Teilbereich des Erfassungsbereichs befindet. Beispielsweise kann vorab ermittelt werden, welcher Bereich der Magnetresonanzanlage als "Gefahrenbereich" anzusehen ist. In diesem Fall wird zusätzlich eine Sicherheitszone um den Gefahrenbereich herum definiert und diese Sicherheitszone als entsprechender Teilbereich des Erfassungsbereichs definiert. In diesem Fall ist die Alarmbedingung also nur dann erfüllt, wenn die erkannte Person sich in die Sicherheitszone hinein bewegt.

Alternativ oder zusätzlich ist es möglich, dass die Alarmbedingung nur dann erfüllt ist, wenn die erkannte Person eine von mindestens einer vorbestimmten Person verschiedene Person ist. In diesem Fall kann das Auslösen des akustischen Signals auf Fälle beschränkt werden, in denen eine Person erkannt wird, die Person der Auswertungseinrichtung aber nicht als autorisiert bekannt ist.

Es ist möglich, dass die Auswertungseinrichtung eine 2D-Auswertung der mittels des Videokamerasystems erfassten Bilder vornimmt. Vorzugsweise nimmt die Auswertungseinrichtung jedoch eine 3D-Auswertung der mittels des Videokamerasystems erfassten Bilder vor. Insbesondere liefert eine 3-D-Auswertung auch eine Tiefeninformation. Dadurch ist oftmals besser auswertbar, in welchem Bereich sich eine Person aufhält und/ oder ob und gegebenenfalls welches weitere Objekt an der Person erkannt wird.

Es ist möglich, dass die Alarmbedingung der Auswertungseinrichtung statisch vorgegeben ist. In einer besonders bevorzugten Ausgestaltung ist die Auswertungseinrichtung jedoch als selbstlernendes System ausgebildet. In diesem Fall ist es insbesondere möglich, dass die Auswertungseinrichtung in dem Fall, dass ihr im Alarmzustand der Beendigungsbefehl vorgegeben wird, die Alarmbedingung dahingehend modifiziert, dass ein Sachverhalt, aufgrund dessen die Alarmbedingung als erfüllt angesehen wurde, aus der Alarmbedingung entfernt wird oder ihm zumindest eine geringere Gewichtung zugeordnet wird.

Die Aufgabe wird weiterhin durch ein Computerprogramm mit den Merkmalen des Anspruchs 9 gelöst. Erfindungsgemäß bewirkt die Abarbeitung des Computerprogramms, dass die Auswertungseinrichtung ein erfindungsgemäßes Überwachungsverfahren ausführt.

Die Aufgabe wird weiterhin durch eine Auswertungseinrichtung mit den Merkmalen des Anspruchs 10 gelöst. Erfindungsgemäß ist die Auswertungseinrichtung mit einer erfindungsgemäßen Auswertungseinrichtung programmiert, so dass sie im Betrieb ein erfindungsgemäßes Überwachungsverfahren ausführt.

Die Aufgabe wird weiterhin durch einen Untersuchungsraum mit den Merkmalen des Anspruchs 11 gelöst. Erfindungsgemäß umfasst bei einem Untersuchungsraum der eingangs genannten Art der Erfassungsbereich alternativ oder zusätzlich zu dem Bereich vor dem Untersuchungsbereich Zugänge zum Untersuchungsraum und ist weiterhin die Auswertungseinrichtung erfindungsgemäß ausgebildet.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die in Verbindung mit den Zeichnungen näher erläutert werden. Hierbei zeigen in schematischer Darstellung:
- FIG 1: einen Untersuchungsraum mit einer darin befindlichen Magnetresonanzanlage von der Seite,
- FIG 2: den Untersuchungsraum von FIG 1 von oben und
- FIG 3 bis 7: Ablaufdiagramme.

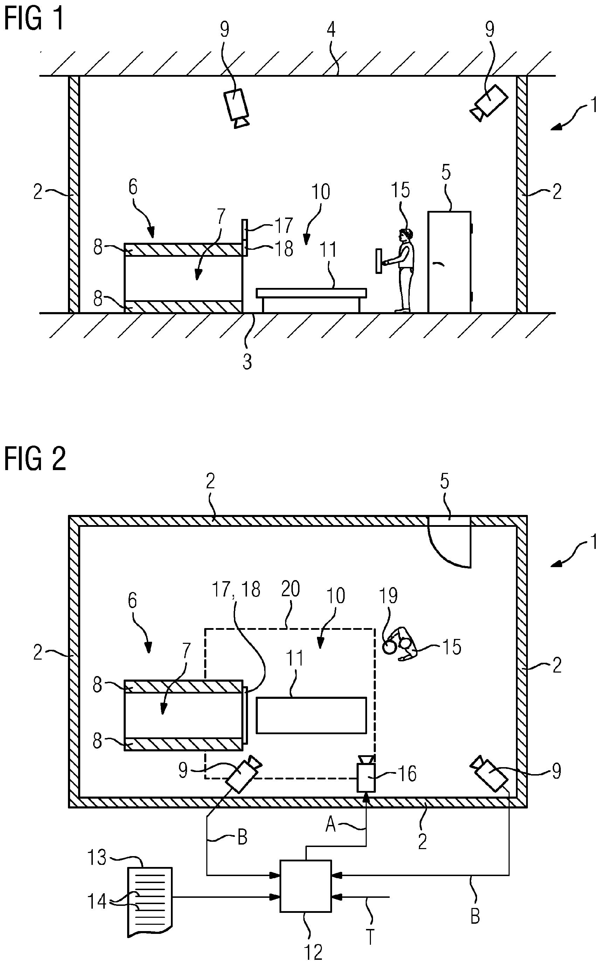

Gemäß den FIG 1 und 2 weist ein Untersuchungsraum 1 Wände 2, einen Boden 3 und eine Decke 4 auf. Die Wände 2 können nach Bedarf durchsichtig oder nicht durchsichtig sein, beispielsweise teilweise verglast. Mindestens eine Wand 2 weist einen Zugang 5 zu dem Untersuchungsraum 1 auf, also eine Türöffnung.

In dem Untersuchungsraum 1 ist eine Magnetresonanzanlage 6 angeordnet. Die Magnetresonanzanlage 6 weist einen Untersuchungsbereich 7 auf, beispielsweise einen Untersuchungstunnel. Der Untersuchungsbereich 7 der Magnetresonanzanlage 6 ist derjenige Bereich, in dem mittels eines Grundmagneten 8 der Magnetresonanzanlage 6 ein zeitlich statisches, örtlich im Wesentlichen homogenes Magnetfeld (in der Praxis meist als B0-Feld bezeichnet) generiert wird. Das B0-Feld weist eine hohe magnetische Feldstärke auf, beispielsweise 1,5 T oder mehr.

Dem Untersuchungsraum 1 ist ein Videokamerasystem 9 zugeordnet. Das Videokamerasystem 9 kann insbesondere innerhalb des Untersuchungsraums 1 angeordnet sein. Mittels des Videokamerasystems 9 werden Bilder B aus einem Erfassungsbereich erfasst. Der Erfassungsbereich umfasst die Zugänge 5 zum Untersuchungsraum 1 und/oder einen Bereich 10 vor dem Untersuchungsbereich 7, beispielsweise den Bereich, in dem sich ein Patiententisch 11 vor dem Einführen des Patiententischs 11 in den Untersuchungsbereich 7 befindet.

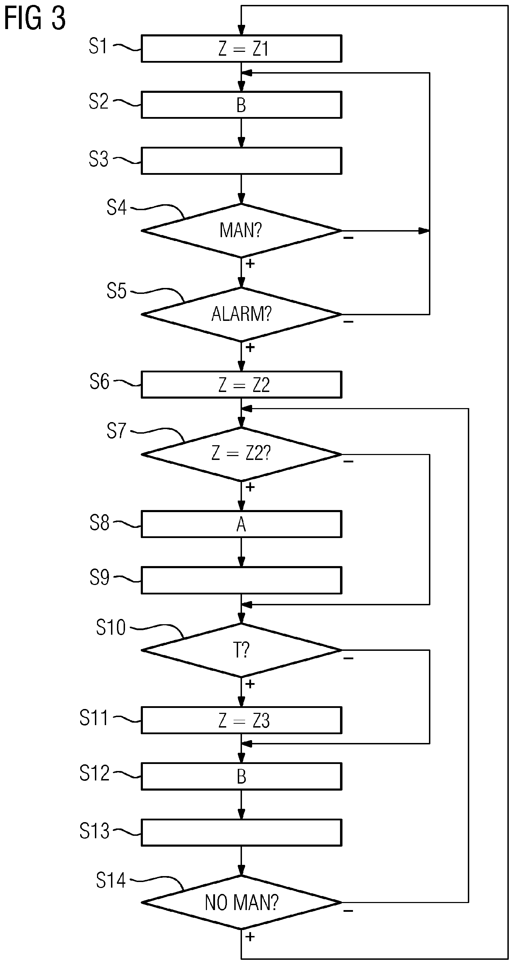

Das Videokamerasystem 9 ist gemäß FIG 2 mit einer Auswertungseinrichtung 12 verbunden. Die Auswertungseinrichtung 12 ist mit einem Computerprogramm 13 programmiert. Das Computerprogramm 13 umfasst Maschinencode 14, der von der Auswertungseinrichtung 12 abarbeitbar ist. Die Abarbeitung des Maschinencodes 14 durch die Auswertungseinrichtung 12 bewirkt, dass die Auswertungseinrichtung 12 im Betrieb ein Überwachungsverfahren ausführt, das nachstehend in Verbindung mit FIG 3 näher erläutert wird.

Zunächst wird ein Zustand Z der Auswertungseinrichtung 12 gemäß FIG 3 in einem Schritt S1 auf einen Bereitschaftszustand Z1 gesetzt. Im Bereitschaftszustand Z1 nimmt die Auswertungseinrichtung 12 von dem Videokamerasystem 9 in einem Schritt S2 eine Gruppe von Bildern B entgegen. Die Gruppe von Bildern B sind diejenigen Bilder B, die von dem Videokamerasystem 9 zu einem bestimmten Zeitpunkt erfasst werden. Es ist möglich, dass die Gruppe nur ein einziges Bild B umfasst. Alternativ kann es sich um mehrere Bilder B handeln.

In einem Schritt S3 nimmt die Auswertungseinrichtung 12 eine Auswertung der entgegengenommenen Gruppe von Bildern B vor. Es ist es möglich, dass die Auswertungseinrichtung 12 eine 2D-Auswertung der Gruppe von Bildern B vornimmt. Vorzugsweise nimmt die Auswertungseinrichtung 12 jedoch eine 3D-Auswertung der Gruppe von Bildern B vor. Beispielsweise kann für mindestens eines der erfassten Bilder B aufgrund einer entsprechenden Projektion eines vorbekannten Musters in den Erfassungsbereich aus dem jeweiligen Bild B eine Tiefeninformation ermittelt werden. Alternativ ist es möglich, durch Korrelation mehrerer Bilder B eine entsprechende dreidimensionale Information zu ermitteln. Die einschlägigen Vorgehensweisen sind Fachleuten allgemein bekannt.

In einem Schritt S4 prüft die Auswertungseinrichtung 12, ob sie im Rahmen der Auswertung eine Person 15 erkennt. Die Erkennung des Schrittes S4 ist hierbei nicht im Sinne einer
Identifizierung der Person 15 zu verstehen. Es geht also nicht darum, ob eine bestimmte Person 15 erkannt wird ("das ist Herr Müller"), sondern darum, ob überhaupt eine Person 15 erkannt wird ("hier ist doch jemand"). Wenn die Auswertungseinrichtung eine Person 15 erkennt, geht die Auswertungseinrichtung 12 zu einem Schritt S5 über. Anderenfalls geht sie zum Schritt S2 zurück.

Im Schritt S5 prüft die Auswertungseinrichtung 12, ob eine Alarmbedingung erfüllt ist. Die Alarmbedingung ist eine vom Erkennen einer Person 15 als solches verschiedene Bedingung. Das Erkennen einer Person 15 als solches löst also nicht stets und unbedingt einen Alarm aus, sondern nur dann, wenn zusätzlich die Alarmbedingung erfüllt ist. Mögliche Ausgestaltungen der Alarmbedingung werden nachstehend in Verbindung mit den weiteren FIG näher erläutert werden. Wenn die Alarmbedingung erfüllt ist, geht die Auswertungseinrichtung 12 zu einem Schritt S6 über. Anderenfalls geht sie zum Schritt S2 zurück.

Im Schritt S6 wird der Zustand Z der Auswertungseinrichtung 12 auf einen Alarmzustand Z2 gesetzt. Die Auswertungseinrichtung 12 geht also in den Alarmzustand Z2 über. In einem nachfolgenden Schritt S7 prüft die Auswertungseinrichtung 12, ob sie sich im Alarmzustand Z2 befindet. Wenn dies der Fall ist, führt die Auswertungseinrichtung 12 einen Schritt S8 aus. Anderenfalls wird der Schritt S8 übersprungen. Im Schritt S8 gibt die Auswertungseinrichtung 12 eine Alarmmeldung A aus. Die Alarmmeldung A umfasst meist ein akustisches Signal. Beispiele geeigneter akustischer Signale sind beispielsweise ein üblicher Alarmton (Hupton, Sirenenton und dergleichen) oder das Ausgeben einer entsprechenden gesprochenen Meldung wie beispielsweise "Vorsicht! Es gibt hier ein sehr starkes Magnetfeld. Gehen Sie sofort zurück". Das Ausgeben des akustischen Signals kann beispielsweise über einen Lautsprecher 16 (siehe FIG 1 und 2) erfolgen. Die akustische Einrichtung,
über die das akustische Signal ausgegeben wird, - beispielsweise der Lautsprecher 16 - ist derart angeordnet, dass das akustische Signal im Untersuchungsraum 1 hörbar ist.

Alternativ oder zusätzlich ist es möglich, dass die Auswertungseinrichtung 12 im Schritt S8 ein optisches Signal ausgibt. Beispielsweise kann die Auswertungseinrichtung 12 ein gelbes oder rotes Blinklicht anschalten oder über eine Anzeigeeinrichtung 17 (siehe FIG 1 und 2) eine mit der gesprochenen Meldung korrespondierende schriftliche Meldung an die Person 15 ausgeben. Die optische Einrichtung, über welche die Auswertungseinrichtung 12 das optische Signal ausgibt, ist derart angeordnet, dass das optische Signal im Untersuchungsraum 1 sichtbar ist. Beispielsweise kann die Anzeigeeinrichtung 17 sich im Bereich unmittelbar vor dem Untersuchungsbereich 7 befinden.

Auf den Schritt S8 kann weiterhin ein Schritt S9 folgen, in dem die Auswertungseinrichtung 12 weitere Maßnahmen ergreift, beispielsweise entsprechende Meldungen an entfernt angeordnete Einrichtungen übermittelt, so dass dort Notfallmaßnahmen eingeleitet werden können.

In einem Schritt S10 prüft die Auswertungseinrichtung 12, ob ihr über eine Mensch-Maschine-Schnittstelle 18 ein Beendigungsbefehl T vorgegeben wird. Wenn die Vorgabe des Beendigungsbefehls T erfolgt, geht die Auswertungseinrichtung 12 zu einem Schritt S11 über. Im Schritt S11 wird der Zustand Z der Auswertungseinrichtung 12 in einen Abschaltzustand Z3 gesetzt. Anderenfalls überspringt die Auswertungseinrichtung 12 den Schritt S11.

Sodann nimmt die Auswertungseinrichtung 12 in einem Schritt S12 von dem Videokamerasystem 9 eine Gruppe von Bildern B entgegen und wertet die entgegengenommene Gruppe von Bildern B in einem Schritt S13 aus. Die Schritte S12 und S13 korrespondieren inhaltlich mit den Schritten S2 und S3.

In einem Schritt S14 prüft die Auswertungseinrichtung 12, ob sie im Rahmen der Auswertung keine Person erkennt, das heißt die Person 15 nicht mehr erkennt. Wenn die Auswertungseinrichtung die Person 15 weiterhin erkennt, geht die Auswertungseinrichtung 12 zum Schritt S7 zurück. Anderenfalls geht sie zum Schritt S1 zurück.

Aufgrund der Prüfung des Schrittes S7 gibt die Auswertungseinrichtung 12 somit das akustische Signal und/oder das optische Signal nicht mehr aus, wenn sie sich im Abschaltzustand Z3 befindet.

Es ist möglich, dass der Schritt S7 als solcher nicht vorhanden ist. In diesem Fall geht die Auswertungseinrichtung 12 vom Schritt S14 aus entweder zum Schritt S9 (falls vorhanden) oder zum Schritt S10 über. In diesem Fall erfolgt auch die Ausgabe der Alarmmeldung A nur einmalig beim Übergang vom Bereitschaftszustand Z1 in den Alarmzustand Z2.

Die Vorgehensweise von FIG 3 wird unabhängig vom Betrieb der Magnetresonanzanlage 6 ausgeführt, vorzugsweise "rund um die Uhr" (im Englischen übliche Bezeichnung: 24/7). Sie wird also insbesondere auch zu Zeiten ausgeführt, zu denen mit der Magnetresonanzanlage 6 überhaupt keine Untersuchungen vorgenommen werden sollen.

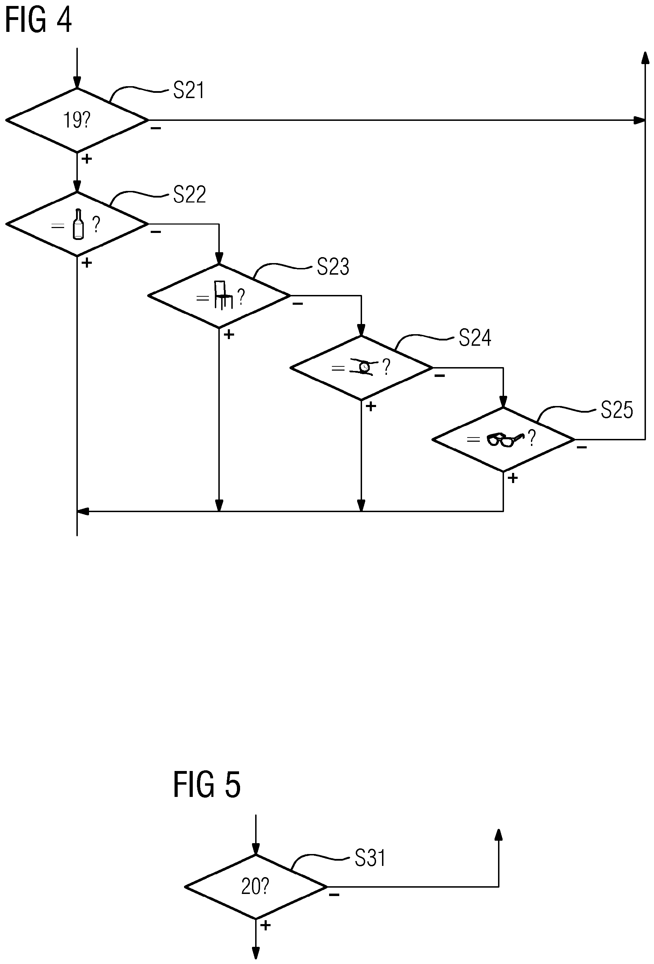

Die Alarmbedingung kann, wie bereits erwähnt, auf unterschiedliche Arten ausgestaltet sein. Nachfolgend werden in Verbindung mit den FIG 4 bis 6 einige mögliche Ausgestaltungen näher erläutert. Die FIG 4 bis 6 zeigen also mögliche Ausgestaltungen des Schrittes S5 von FIG 3.

Gemäß FIG 4 prüft die Auswertungseinrichtung 12 in einer möglichen Ausgestaltung in einem Schritt S21, ob sie zusätzlich zu der erkannten Person 15 (wobei die Bekleidung der Person 15 als Bestandteil der Person 15 angesehen wird) an der Person 15 ein weiteres Objekt 19 erkennt. Es ist möglich, dass die Prüfung des Schrittes S21 die einzige Prüfung ist. In diesem Fall entscheidet das Erkennen des weiteren Objektes 19 als solches darüber, ob die Alarmbedingung erfüllt ist oder nicht. Das Erkennen des weiteren Objekts 19 ist in diesem Fall also nicht nur eine notwendige, sondern zugleich auch eine hinreichende Bedingung dafür, die Alarmbedingung als erfüllt anzusehen. Vorzugsweise ist die Alarmbedingung entsprechend der Darstellung in FIG 4 jedoch nur dann erfüllt, wenn das erkannte Objekt 19 eine hinreichende Übereinstimmung mit mindestens einem vorbestimmten Objekttyp aufweist. Beispielsweise kann entsprechend der Darstellung in FIG 4 nacheinander geprüft werden, ob das erkannte Objekt 19 eine hinreichende Ähnlichkeit mit einer Flasche, einem Stuhl, einer Armbanduhr und/oder einer Brille aufweist. Die entsprechenden Prüfungen sind in FIG 4 in Schritten S22 bis S25 dargestellt. Selbstverständlich müssen nicht alle dargestellten Prüfungen vorgenommen werden. Weiterhin können nach Bedarf auch andere oder zusätzliche Prüfungen vorgenommen werden. Prüfverfahren, mittels derer entsprechende Objekttypen erkannt werden können, sind Fachleuten allgemein bekannt. Sie müssen daher nicht näher erläutert werden.

Weiterhin ist es entsprechend der Darstellung in FIG 5 beispielsweise möglich, dass die Auswertungseinrichtung 12 in einem Schritt S31 prüft, ob die erkannte Person 15 sich in einem vorbestimmten Teilbereich 20 des Erfassungsbereichs befindet. Beispielsweise ist es möglich, dass die Alarmbedingung noch nicht erfüllt ist, solange die erkannte Person 15 hinreichend weit von der Magnetresonanzanlage 6 und insbesondere dem Untersuchungsbereich 7 entfernt ist. Wenn die erkannte Person 15 sich hingegen in den vorbestimmten Teilbereich 20 hinein bewegt kann - mit oder ohne Prüfung weiterer Bedingungen - die Alarmbedingung als erfüllt angenommen werden und somit der Alarm ausgelöst werden. Die Prüfung gemäß FIG 5 kann nach Bedarf mit der Prüfung gemäß FIG 4 kombiniert werden. Beispielsweise ist es möglich, die beiden Prüfungen unabhängig voneinander durchzuführen, so dass die Alarmbedingung bereits erfüllt ist, sowie eine der beiden Prüfungen positiv ist. Alternativ ist es möglich, die beiden Prüfungen im Sinne einer UND-Verknüpfung miteinander zu kombinieren, so dass die Alarmbedingung erst dann erfüllt ist, wenn zum einen das zusätzliche Objekt 19 (gegebenenfalls einschließlich Objekttyp) erkannt wurde und weiterhin die Person 15 sich in dem vorbestimmten Teilbereich 20 befindet. Beispielsweise kann zu diesem Zweck die Vorgehensweise von FIG 5 als Vorprüfung vor der Prüfung gemäß FIG 4 ausgeführt werden.

Es ist auch möglich, die Prüfung von FIG 5 mehrfach gestuft vorzunehmen, beispielsweise also mehrere ineinander verschachtelte Teilbereiche zu definieren, und eine umso intensivere Warnung auszugeben, je weiter die Person 15 voranschreitet.

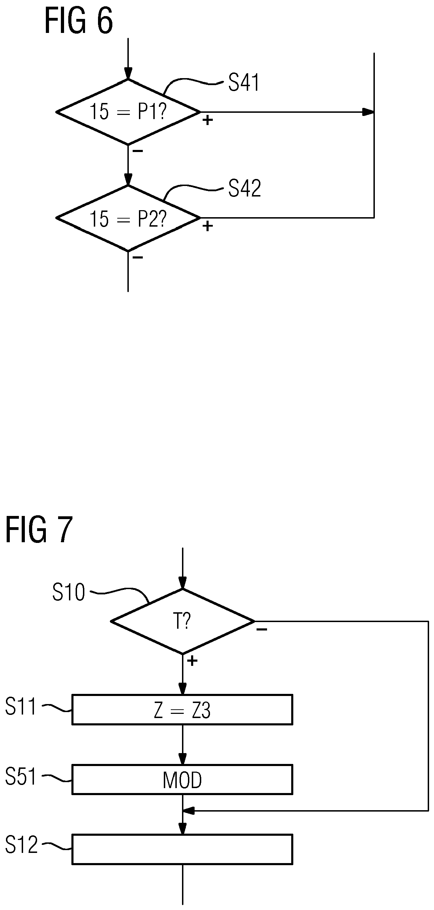

Weiterhin ist es entsprechend der Darstellung in FIG 6 möglich, dass die Auswertungseinrichtung 12 in Schritten S41 und S42 prüft, ob sie die erkannte Person 15 identifizieren kann, ob sie also beispielsweise im Schritt S41 erkennen kann, dass es sich bei der erkannten Person 15 um eine erste vorbekannte Person P1 ("das ist Herr Müller") handelt, oder im Schritt S42 erkennen kann, dass es sich bei der erkannten Person 15 um eine zweite vorbekannte Person P2 ("das ist Frau Braun") handelt. Diese Vorgehensweise kann selbstverständlich auch auf mehr als zwei Personen erweitert werden. In diesem Fall kann die Alarmbedingung beispielsweise erfüllt sein, wenn die Auswertungseinrichtung 12 die erkannte Person 15 nicht identifizieren kann. In diesem Fall konnte die Auswertungseinrichtung 12 im Rahmen der Auswertung des Schrittes S3 also zwar erkennen, dass eine Person 15 vorhanden ist, aber nicht, wer die Person 15 ist.

Die Personenidentifizierung kann nach Bedarf ausgestaltet sein. Insbesondere sind Fachleuten entsprechende Verfahren zur Gesichtserkennung allgemein bekannt. Auch kann - alternativ oder zusätzlich - eine Auswertung der Statur der Person 15 erfolgen.

Die Vorgehensweise von FIG 6 kann im Falle der gleichzeitigen Erkennung mehrerer Personen 15 dahingehend ausgestaltet sein, dass die Alarmbedingung als erfüllt angesehen wird, sowie die Auswertungseinrichtung 12 eine der Personen 15 nicht identifizieren kann. Vorzugsweise aber wird im Falle der Erkennung mehrerer Personen 15 die Alarmbedingung nur dann als erfüllt angesehen, wenn die Auswertungseinrichtung 12 keine der erkannten Personen 15 identifizieren kann. Die Vorgehensweise von FIG 6 kann weiterhin nach Bedarf - sei es als Alternative, sei es als zusätzliche Bedingung - mit den Vorgehensweisen von FIG 4, FIG 5 bzw. FIG 4 und 5 kombiniert werden.

Im Regelfall ist die Alarmbedingung aus Sicht der Auswertungseinrichtung 12 statisch. Sie ist ihr also von außen vorgegeben und wird von der Auswertungseinrichtung 12 nicht geändert. Es ist jedoch möglich, dass die Auswertungseinrichtung 12 insofern selbstlernend ist, als sie selbstständig "unkritische" Situationen lernen kann. Dies wird nachfolgend in Verbindung mit FIG 7 näher erläutert.

FIG 7 geht aus von FIG 3. Es wird daher auf die obigen Ausführungen zu FIG 3 (und auch die Ausgestaltungen gemäß den FIG 4 bis 6 verwiesen. Zusätzlich ist jedoch ein Schritt S51 vorhanden. Der Schritt S51 ist dem Schritt S11 nachgeordnet. Er wird also ausgeführt, wenn der Auswertungseinrichtung 12 im Alarmzustand Z2 der Beendigungsbefehl T vorgegeben wird. Im Schritt S51 modifiziert die Auswertungseinrichtung 12 die Alarmbedingung. Die Modifikation erfolgt dahingehend, dass der im Rahmen der Auswertung der Gruppe von Bildern B erkannte Sachverhalt, aufgrund dessen die Alarmbedingung als erfüllt angesehen wurde, aus der Alarmbedingung entfernt wird. Zumindest wird diesem Sachverhalt im Schritt S51 eine geringere Gewichtung zugeordnet. Im erstgenannten Fall wird der Sachverhalt sofort aus der Alarmbedingung entfernt. Im letztgenannten Fall wird der Sachverhalt immer schwächer gewichtet, so dass er im Ergebnis nach mehreren Iterationen aus der Alarmbedingung entfernt wird.

Die vorliegende Erfindung weist viele Vorteile auf. Insbesondere ist es möglich, Unfälle, die auf versehentliches Missachten der Sicherheitsvorschriften zurückzuführen sind, nahezu vollständig zu vermeiden. Die Betriebssicherheit kann deutlich erhöht werden.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Überwachungsverfahren für einen Untersuchungsraum (1), in dem eine einen Untersuchungsbereich (7), insbesondere einen Untersuchungstunnel, aufweisende Magnetresonanzanlage (6) angeordnet ist, wobei eine Auswertungseinrichtung (12)
- unabhängig vom Betrieb der Magnetresonanzanlage (6) von einem Videokamerasystem (9) aus einem Erfassungsbereich Bilder (B) entgegennimmt, wobei der Erfassungsbereich Zugänge (5) zum Untersuchungsraum (7) und/oder einen Bereich (10) vor dem Untersuchungsbereich (7) umfasst,
- immer dann, wenn sie in einem Bereitschaftszustand (Z1) in den entgegengenommenen Bildern (B) eine Person (15) erkennt, prüft, ob eine vom Erkennen einer Person (15) als solches verschiedene Alarmbedingung erfüllt ist,
- immer dann, wenn die Alarmbedingung erfüllt ist, in einen Alarmzustand (Z2) übergeht, in dem sie zumindest einmalig zumindest ein im Untersuchungsraum (1) hörbares akustisches Signal und/oder ein im Untersuchungsraum (1) sichtbares optisches Signal ausgibt, und anderenfalls den Bereitschaftszustand (Z1) beibehält,
- im Alarmzustand (Z2) prüft, ob ihr über eine Mensch-Maschine-Schnittstelle (18) ein Beendigungsbefehl (T) vorgegeben wird,
- bei Vorgabe des Beendigungsbefehls (T) in einen Abschaltzustand (Z3) übergeht, in dem sie das akustische Signal und/ oder das optische Signal nicht mehr ausgibt,
- im Alarmzustand (Z2) und im Abschaltzustand (Z3) prüft, ob sie in den Bildern (B) weiterhin die Person (15) erkennt,
- immer dann, wenn sie im Alarmzustand (Z2) und im Abschaltzustand (Z3) weiterhin die Person (15) erkennt, den momentanen Zustand (Z2, Z3) beibehält und anderenfalls in den Bereitschaftszustand (Z1) übergeht.

2. Überwachungsverfahren nach Anspruch 1,
**dadurch gekennzeichnet,**
**dass** die Alarmbedingung nur dann erfüllt ist, wenn die Auswertungseinrichtung (12) in den entgegengenommenen Bildern (B) zusätzlich zu der Person (15) an der Person (15) ein von der Person (15) und dessen Bekleidung verschiedenes Objekt (19) erkennt.

3. Überwachungsverfahren nach Anspruch 2,
**dadurch gekennzeichnet,**
**dass** die Alarmbedingung nur dann erfüllt ist, wenn das erkannte Objekt (19) eine hinreichende Übereinstimmung mit mindestens einem vorbestimmten Objekttyp aufweist.

4. Überwachungsverfahren nach Anspruch 3,
**dadurch gekennzeichnet,**
**dass** die Objekttypen eine Flasche, einen Stuhl, eine Armbanduhr und/oder eine Brille umfassen.

5. Überwachungsverfahren nach einem der obigen Ansprüche,
**dadurch gekennzeichnet,**
**dass** die Alarmbedingung nur dann erfüllt ist, wenn die erkannte Person (15) sich in einem vorbestimmten Teilbereich (20) des Erfassungsbereichs befindet.

6. Überwachungsverfahren nach einem der obigen Ansprüche,
**dadurch gekennzeichnet,**
**dass** die Alarmbedingung nur dann erfüllt ist, wenn die erkannte Person (15) eine von mindestens einer vorbestimmten Person (P1, P2) verschiedene Person ist.

7. Überwachungsverfahren nach einem der obigen Ansprüche,
**dadurch gekennzeichnet,**
**dass** die Auswertungseinrichtung (12) eine 3D-Auswertung der mittels des Videokamerasystems (9) erfassten Bilder (B) vornimmt.

8. Überwachungsverfahren nach einem der obigen Ansprüche,
**dadurch gekennzeichnet,**
**dass** die Auswertungseinrichtung (12) in dem Fall, dass ihr im Alarmzustand (Z2) der Beendigungsbefehl (T) vorgegeben wird, die Alarmbedingung dahingehend modifiziert, dass ein Sachverhalt, aufgrund dessen die Alarmbedingung als erfüllt angesehen wurde, aus der Alarmbedingung entfernt wird oder ihm zumindest eine geringere Gewichtung zugeordnet wird.

9. Computerprogramm, das Maschinencode (14) umfasst, der von einer Auswertungseinrichtung (12) abarbeitbar ist, wobei die Abarbeitung des Maschinencodes (14) durch die Auswertungseinrichtung (12) bewirkt, dass die Auswertungseinrichtung (12) ein Überwachungsverfahren nach einem der obigen Ansprüche ausführt.

10. Auswertungseinrichtung, wobei die Auswertungseinrichtung mit einem Computerprogramm (13) nach Anspruch 9 programmiert ist, so dass die Auswertungseinrichtung im Betrieb ein Überwachungsverfahren nach einem der Ansprüche 1 bis 8 ausführt.

11. Untersuchungsraum,
- wobei in dem Untersuchungsraum eine einen Untersuchungsbereich (7), insbesondere einen Untersuchungstunnel, aufweisende Magnetresonanzanlage (6) angeordnet ist,
- wobei dem Untersuchungsraum ein Videokamerasystem (9) zugeordnet ist, mittels dessen aus einem Erfassungsbereich Bilder (B) erfassbar sind,
- wobei der Erfassungsbereich Zugänge (5) zum Untersuchungsraum und/oder einen Bereich (10) vor dem Untersuchungsbereich (7) umfasst,
- wobei das Videokamerasystem (9) mit einer Auswertungseinrichtung (12) verbunden ist,
- wobei die Auswertungseinrichtung (12) gemäß Anspruch 10 ausgebildet ist.

## Geänderte Patentansprüche

### Geänderte Patentansprüche gemäss Regel 137(2) EPÜ.

1. Überwachungsverfahren für einen Untersuchungsraum (1), in dem eine einen Untersuchungsbereich (7), insbesondere einen Untersuchungstunnel, aufweisende Magnetresonanzanlage (6) angeordnet ist, wobei eine Auswertungseinrichtung (12)
- unabhängig vom Betrieb der Magnetresonanzanlage (6) von einem Videokamerasystem (9) aus einem Erfassungsbereich Bilder (B) entgegennimmt, wobei der Erfassungsbereich Zugänge (5) zum Untersuchungsraum (7) und/oder einen Bereich (10) vor dem Untersuchungsbereich (7) umfasst,
- immer dann, wenn sie in einem Bereitschaftszustand (Z1) in den entgegengenommenen Bildern (B) eine Person (15) erkennt, prüft, ob eine vom Erkennen einer Person (15) als solches verschiedene Alarmbedingung erfüllt ist,
- immer dann, wenn die Alarmbedingung erfüllt ist, in einen Alarmzustand (Z2) übergeht, in dem sie zumindest einmalig zumindest ein im Untersuchungsraum (1) hörbares akustisches Signal und/oder ein im Untersuchungsraum (1) sichtbares optisches Signal ausgibt, und anderenfalls den Bereitschaftszustand (Z1) beibehält,
- im Alarmzustand (Z2) prüft, ob ihr über eine Mensch-Maschine-Schnittstelle (18) ein Beendigungsbefehl (T) vorgegeben wird,
- bei Vorgabe des Beendigungsbefehls (T) in einen Abschaltzustand (Z3) übergeht, in dem sie das akustische Signal und/ oder das optische Signal nicht mehr ausgibt,
- im Alarmzustand (Z2) und im Abschaltzustand (Z3) prüft, ob sie in den Bildern (B) weiterhin die Person (15) erkennt,
- immer dann, wenn sie im Alarmzustand (Z2) und im Abschaltzustand (Z3) weiterhin die Person (15) erkennt, den momentanen Zustand (Z2, Z3) beibehält und anderenfalls in den Bereitschaftszustand (Z1) übergeht.

2. Überwachungsverfahren nach Anspruch 1,
**dadurch gekennzeichnet,**
**dass** die Alarmbedingung nur dann erfüllt ist, wenn die Auswertungseinrichtung (12) in den entgegengenommenen Bildern (B) zusätzlich zu der Person (15) an der Person (15) ein von der Person (15) und dessen Bekleidung verschiedenes Objekt (19) erkennt.

3. Überwachungsverfahren nach Anspruch 2,
**dadurch gekennzeichnet,**
**dass** die Alarmbedingung nur dann erfüllt ist, wenn das erkannte Objekt (19) eine hinreichende Übereinstimmung mit mindestens einem vorbestimmten Objekttyp aufweist.

4. Überwachungsverfahren nach Anspruch 3,
**dadurch gekennzeichnet,**
**dass** die Objekttypen eine Flasche, einen Stuhl, eine Armbanduhr und/oder eine Brille umfassen.

5. Überwachungsverfahren nach einem der obigen Ansprüche,
**dadurch gekennzeichnet,**
**dass** die Alarmbedingung nur dann erfüllt ist, wenn die erkannte Person (15) sich in einem vorbestimmten Teilbereich (20) des Erfassungsbereichs befindet.

6. Überwachungsverfahren nach einem der obigen Ansprüche,
**dadurch gekennzeichnet,**
**dass** die Alarmbedingung nur dann erfüllt ist, wenn die erkannte Person (15) eine von mindestens einer vorbestimmten Person (P1, P2) verschiedene Person ist.

7. Überwachungsverfahren nach einem der obigen Ansprüche,
**dadurch gekennzeichnet,**
**dass** die Auswertungseinrichtung (12) eine 3D-Auswertung der mittels des Videokamerasystems (9) erfassten Bilder (B) vornimmt.

8. Überwachungsverfahren nach einem der obigen Ansprüche,
**dadurch gekennzeichnet,**
**dass** die Auswertungseinrichtung (12) in dem Fall, dass ihr im Alarmzustand (Z2) der Beendigungsbefehl (T) vorgegeben wird, die Alarmbedingung dahingehend modifiziert, dass ein Sachverhalt, aufgrund dessen die Alarmbedingung als erfüllt angesehen wurde, aus der Alarmbedingung entfernt wird oder ihm zumindest eine geringere Gewichtung zugeordnet wird.

9. Computerprogramm, das Maschinencode (14) umfasst, der von einer Auswertungseinrichtung (12) abarbeitbar ist, wobei die Abarbeitung des Maschinencodes (14) durch die Auswertungseinrichtung (12) bewirkt, dass die Auswertungseinrichtung (12) ein Überwachungsverfahren nach einem der obigen Ansprüche ausführt.

10. Auswertungseinrichtung, wobei die Auswertungseinrichtung mit einem Computerprogramm (13) nach Anspruch 9 programmiert ist, so dass die Auswertungseinrichtung im Betrieb ein Überwachungsverfahren nach einem der Ansprüche 1 bis 8 ausführt.

11. Untersuchungsraum,
- wobei in dem Untersuchungsraum eine einen Untersuchungsbereich (7), insbesondere einen Untersuchungstunnel, aufweisende Magnetresonanzanlage (6) angeordnet ist,
- wobei dem Untersuchungsraum ein Videokamerasystem (9) zugeordnet ist, mittels dessen aus einem Erfassungsbereich Bilder (B) erfassbar sind,
- wobei der Erfassungsbereich Zugänge (5) zum Untersuchungsraum und/oder einen Bereich (10) vor dem Untersuchungsbereich (7) umfasst,
- wobei das Videokamerasystem (9) mit einer Auswertungseinrichtung (12) verbunden ist,
- wobei die Auswertungseinrichtung (12) gemäß Anspruch 10 ausgebildet ist.
